# EUROPEAN PATENT SPECIFICATION

(11) **EP 1 890 588 B1**
(45) Date of publication and mention of the grant of the patent: **16.08.2017**
(21) Application number: 06703880.2
(22) Date of filing: 26.01.2006
(51) Int. Cl.: A61B 5/00, A61M 5/142

(54) **AMBULATORY MEDICAL DEVICE AND METHOD OF COMMUNICATION BETWEEN MEDICAL DEVICES**
AMBULANTE MEDIZINISCHE VORRICHTUNG UND VERFAHREN ZUR KOMMUNIKATION ZWISCHEN MEDIZINISCHEN VORRICHTUNGEN
DISPOSITIF MEDICAL AMBULATOIRE ET PROCEDE DE COMMUNICATION ENTRE DES DISPOSITIFS MEDICAUX

(30) Priority: 02.02.2005 EP 05002074
(43) Date of publication of application: 27.02.2008
(60) Divisional application: 17180269.7
(73) Proprietor: F. Hoffmann-La Roche AG, 4070 Basel (CH); Roche Diabetes Care GmbH, 68305 Mannheim (DE)
(72) Inventor: ESSENPREIS, Matthias, CH-3400 Burgdorf (CH); HAUETER, Ulrich, CH-3506 Grosshoechstetten (CH); BERNINI, Nicole, CH-3423 Ersigen (CH); FANKHAUSER, Sybille, CH-4542 Luterbach (CH); LA BASTIDE, Sebastiaan, CH-3074 Muri Bei Bern (CH); MEYER OLDEN, Gunnar, CH-3400 Burgdorf (CH); SCHOEMAKER, Michael, 68183 Mannheim (DE); HEATON, Kelly, CH-3423 Ersigen (CH); JECKELMANN, Joel, CH-1752 Villars-sur-Glane (CH)
(74) Representative: Rentsch Partner AG
(86) International application number: PCT/EP2006/000663
(87) International publication number: WO 2006/081975

(56) References cited:
- EP-A- 0 098 592
- EP-A1- 1 338 295
- US-A- 5 507 288
- US-A1- 2002 002 326
- US-A1- 2002 049 389
- US-A1- 2002 065 453
- US-A1- 2002 143 241
- US-A1- 2003 065 536
- US-A1- 2003 069 509
- US-A1- 2004 167 464
- US-A1- 2004 225 338
- US-A1- 2007 282 177

## Description

### Technical field of the invention

The present invention relates to an ambulatory medical device and a method of communication for medical devices.

### Background Art

Ambulatory medical devices for the treatment of diabetes include e.g. extra corporal insulin pumps and blood glucose measuring devices such as e.g. hand held glucose meters. Insulin pumps allow a good control of blood glucose concentrations by continuously infusing a basic amount of insulin in a human body (basal insulin rate) and manually controlled additional "meal bolus" insulin quantities thereby reflecting the insulin secretion by the pancreas. Furthermore, the development of continuous glucose sensors will allow to measure in vivo glucose concentrations over the whole day. The measured glucose date can be used to adjust the diabetes therapy to individual needs.

In order to improve the treatment of diabetes it is important to provide means and methods to transfer data between medical devices in a quality assuring way

It is therefore the aim of the present invention to provide a medical device allowing a controlled data transfer between medical devices and a method of controlled data transfer.

### Disclosure of the invention

In a first aspect, the present invention relates to a medical device comprising a module for communication with at least a second medical device wherein the module for communication in said medical device is adapted to be activated by a value of a physiological parameter of an animal.

The inventive medical device preferably comprises a telemetry system for wireless communication, preferably a telemetry system for RF communication.

### Disclosure of the invention

The present invention relates to a first medical device comprising a module for communication with at least a second medical device wherein the module for communication in said first medical device is adapted to be activated by a value of an analyte concentration of an animal.

The first medical device preferably comprises a telemetry system for wireless communication, preferably a telemetry system for RF communication.

Preferably the first medical device is selected from the group consisting of a glucose measuring device such as e.g. a hand held glucose meter and a strip based glucose meter or combinations thereof.

In a preferred embodiment of the present invention the analyte concentration is a blood glucose concentration.

Preferably, the first medical device comprises an electrochemical or photometric module for measuring blood glucose. Suitable medical devices are e.g. strip based glucose meters such as AccuChek Compact.

The present invention relates to a system of medical devices. Said system comprises the first medical device as described in the previous section and the second medical device capable to communicate with said first medical device.

In a preferred embodiment the second medical device is selected from the group consisting of a continuous analyte or vital sign sensor and a continuous glucose sensor.

In a further preferred embodiment the first medical device and the at least second medical device comprise a telemetry system for wireless communication, preferably a telemetry system for RF communication.

In a further aspect, the present invention relates to a method of communication between a first medical device and at least a second medical device wherein the communication between said medical devices is enabled and/or activated by a value of an analyte concentration of an animal.

The communication between the at least two medical devices is enabled and/or activated for a predetermined time. Time duration can be fixed, random or dependent on the analyte concentration enabling and/or activating the communication or other physiological parameters of the animal body.

The physiological parameter is preferably selected from the group consisting of an analyte concentration, a physiological characteristic like conductivity of an animal, a physiological vital sign like heart or breath rate, temperature, movement, air- or structure-borne sound, ECG (electrocardiogram) and the like. Preferably the analyte concentration is a blood glucose concentration.

In a further preferred embodiment, the first medical device is selected from the group consisting of a a glucose measuring device and a strip based glucose meter.

The second medical device is preferably selected from the group consisting of an analyte sensor, preferably a continuous analyte sensor, and a continuous glucose sensor.

In a preferred embodiment, the communication between said medical devices is a wireless communication, preferably a RF communication.

### Detailed description of the invention

In one aspect, the present invention relates to a novel method for controlling and/or enabling communication between medical devices, in particular medical sensory devices such as continuous glucose sensors and/or therapeutic devices such as insulin pumps and/or diagnostic medical devices such as glucose meters.

For example, the communication between a continuous glucose sensor applied to a human body and a blood glucose meter can only be established when a blood glucose measurement has been made in the blood glucose meter. The generation of the blood glucose value in the blood glucose meter enables and/or activates communication between the two devices for a specified time limit. During the time window data can be transferred from the sensor to the glucose meter and/or commands from the glucose meter can be sent to the sensor. After expiration of a time limit, communication between the two devices is deactivated. In order to establish a further communication, the communication link between the two devices has to be activated by generating a further blood glucose value in the glucose meter.

The term "generation of a value" as it is used herein encompasses any method or procedure for the determination of physiological parameters such as methods for the measurement of analyte values, in particular blood glucose values. Suitable methods for the determination of blood glucose values are e.g. electrochemical methods and photometric methods which are known to a person skilled in the art.

The dependence of the communication link between medical devices on an actual analyte value ensures the quality of the data transmitted from the medical sensory device and/or medical therapeutical device to the medical diagnostic device.

The data transferred from the sensor to the diagnostic device can be stored on the diagnostic device e.g. a glucose meter and be transferred to a third device such as a PDA or a computer, for further processing and/or analysis. By means of suitable software the data can be analyzed and used for e.g. bolus recommendation or adjustment of basal insulin rates for patients using an extra- or intra corporal insulin pump.

The communication link between the diagnostic device and a third device e.g. a computer, does not need activation by generation of a blood glucose value in said diagnostic device.

In a preferred embodiment, the present invention relates to a method of communication between a diagnostic medical device, preferably a blood glucose meter, and an infusion pump, preferably an extra corporal insulin pump. In this specific embodiment, the diagnostic medical device is used as a remote control to control the function of the infusion pump. After a blood glucose value has been generated in the blood glucose meter a communication link between meter and pump is enabled and/or activated for a defined time and commands can be transferred from the remote control i.e. the glucose meter, to the pump. It is as well possible to transfer data stored on the pump to the diagnostic device during the communication time window.

When the remote control of the infusion pump does not comprise a module for measuring blood glucose concentration, the communication between pump and remote control is activated by entering a current blood glucose value measured in a blood glucose meter in the remote control. The value can e.g. be entered using buttons of the remote control or can be transferred via a wireless or wired connection to the glucose meter. After the blood glucose value has been entered in the remote control, a communication link between remote control and infusion pump is established, preferably for a predetermined time span. After expiration of the time span, the communication is interrupted and no data exchange between the two devices is anymore possible. A further round of communication needs a new activation of the communication by entering a new, current blood glucose value in the remote control. The term remote control as used herein encompasses PDA, smart phones, a handy and pump specific remote controls.
The data transfer between the medical devices can be performed using known technologies and comprise wired connections as well as wireless communications. These technologies are known to a person skilled in the art. The preferred communication is a wireless communication, more preferably RF communication.

The data transfer between the devices can be encrypted in order to ensure that non-authorized third parties do not gain access to personal data of patients. Methods of encrypting data are known to a person skilled in the art.

In a further preferred embodiment, the communication between the medical devices is activated by a manipulation on the second medical device e.g. an insulin pump, such as pressing a button or lever, inserting a battery, using the touch screen, shaking, bumping or squeezing or the like.

In the following paragraph a preferred embodiment of the present invention is described.

The preferred system of medical devices comprises a continuous glucose sensor device which is placed on a human body in order to measure glucose value in interstitial fluid and a glucose meter. The sensor device comprises an electrochemical glucose sensor measuring the glucose concentration in the interstitial tissue in a predetermined manner. The sensor device further comprises an extra corporal part including processor means for controlling the sensor, memory for storing measured glucose values and a telemetry system for transmitting the data to a glucose meter, preferably a strip-based glucose meter. The glucose values stored on the sensor device are then transferred to a glucose meter via the telemetry system.

The communication between the two devices i.e. the wireless link, is established and/or activated by measuring the glucose concentration in a blood sample of a patient using the glucose meter, preferably a strip-based glucose meter. When a strip-based glucose meter is used, the patient inserts a strip in the glucose meter and puts a droplet of blood on the strip. The glucose meter measures and indicates on its display the blood glucose value. After measurement of the blood glucose value, the communication link can then be activated/established either by pressing a button on the glucose meter e.g. an activation button or by a direct electronic link to the processor controlling the glucose telemetry system such that the completion of the blood glucose measurement automatically activates the wireless link between the devices.

The communication link is then established and a data transfer between the medical devices is possible for a defined time span. After expiration of the defined time span the communication link is deactivated and no further data/commands can be transmitted between the medical devices. A new blood glucose measurement in the glucose meter is then necessary to open a new wireless link between the medical devices.

In a further aspect, the present invention relates to a method of data processing or data use. Said method is characterised in that data processing or data use is only possible after activation by a value of a physiological parameter. The method is preferably used for the processing of medical data such as data measured by a sensor applied on a human body.

In a preferred embodiment, the method is used for the processing of medical data which have been measured by a sensor device applied on a human being, preferably a continuous glucose sensor. The data are then e.g. transferred to a diagnostic medical device, preferably a blood glucose meter. The data are preferably transferred via a wireless link from the sensor device to the diagnostic device. In this case, each of the at least two medical devices comprises a telemetry system for wireless communication. The wireless communication can be bidirectional or unidirectional.

There is preferably a permanent communication link between said two medical devices but the data stored e.g. in the memory of the medical sensor device and transferred to the diagnostic device can only be further processed on the diagnostic device after the processing has been activated by a value of a physiological parameter. After activation by a value of a physiological parameter, preferably a blood glucose value, the data stored in the memory of the diagnostic device can be processed or used. For example, data are transferred from a continuous glucose sensor to a glucose meter and stored in the memory of the glucose meter. The further processing of these data is then only possible after activation of the processing by a value of a physiological parameter, preferably a blood glucose value.

In a preferred embodiment, the processing of the data is only possible for a limited time span after activation by a value of a physiological parameter. When the defined time span for data processing has lapsed, no further data processing is possible without a new activation by a value of a physiological parameter.

In a further aspect, the present invention relates to a medical device comprising a module for data processing which is adapted to be activated by a value of a physiological parameter. Preferably said module comprises a microprocessor with a memory for storing data.

Said medical device is preferably a blood glucose meter. Preferably, the processing of data stored in the memory of the blood glucose meter is either activated by pressing a button on the glucose meter (an activation button) or by a direct electronic link to the processor controlling the data processing module/system such that the completion of the blood glucose measurement automatically activates data processing

The terms "data processing" or "data use" as they are used herein refer to any manipulation of data and comprise analysis of data, presentation of data, interpretation of data and indication of data.

## Claims

1. A system of medical devices comprising:
a) a first medical device, the first medical device being an analyte measuring device, and
b) a second medical device capable to communicate with said first medical device,
wherein the second medical device is an analyte sensor,
wherein said first medical device comprises a module for communication with the second medical device, wherein the module for communication in the first medical device is adapted to be activated by a value of an analyte concentration of an animal, wherein the generation of the value of the analyte concentration activates the communication between the two devices for a specified time limit and data can be transferred from the second medical device to the first medical device and/or commands from the first medical device can be sent to the second medical device, wherein after expiration of the time limit, the communication link between the two devices is deactivated, wherein in order to establish a further communication, the communication link between the two devices has to be activated by generating a further value of an analyte concentration of the animal, and wherein the first medical device is configured to send commands to the second medical device to control at least partially the second medical device.

2. The system of claim 1, wherein the module for communication is a telemetry system for wireless communication, preferably a telemetry system for RF communication.

3. The system of claim 1 or 2, wherein the first medical device is selected from the group consisting of a glucose measuring device and a strip based glucose measuring device.

4. The system of claim 1, wherein the analyte concentration is a blood glucose concentration.

5. The system of claim 4, wherein the first medical device comprises an electrochemical module for measuring blood glucose.

6. The system of claim 4, wherein the first medical device comprises a photometric module for measuring blood glucose.

7. The system of claim 1, wherein the second medical device is selected from the group consisting of a continuous analyte sensor and a continuous glucose sensor.

8. The system of claim 1 or 7, wherein the first medical device and the second medical device comprise a telemetry system for wireless communication, preferably a telemetry system for RF communication.

9. A method of communication between a first medical device and a second medical device, the first medical device being an analyte measuring device, wherein the second medical device is an analyte sensor, wherein said first medical device comprises a module for communication with the second medical device, wherein the module for communication in the first medical device is adapted to be activated by a value of an analyte concentration of an animal, wherein the generation of the value of the analyte concentration of the animal activates the communication link between the two devices for a specified time limit and enables that data can be transferred from the second medical device to the first medical device and/or commands from the first medical device can be sent to the second medical device, wherein after expiration of the time limit, the communication link between the two devices is deactivated, wherein in order to establish a further communication, the communication link between the two devices has to be activated by generating a further value of an analyte concentration of the animal, and wherein the first medical device sends commands to the second medical device to control at least partially the second medical device.

10. The method of claim 9, wherein the time window is established according to a value of a physiological characteristic of an animal or according to a set of data gained during previous time periods.

11. The method of claims 9 or 10, wherein the first medical device is selected from the group consisting of a glucose measuring device and a strip-based glucose measuring device.

12. The method of one of claims 9 to 11, wherein the second medical device is selected from the group consisting of a continuous analyte sensor and a continuous glucose sensor.

13. The method of one of claims 9 to 12, wherein the communication between said medical devices is a wireless communication, preferably a RF communication.

## Patentansprüche

1. System von medizinischen Vorrichtungen, umfassend:
a) eine erste medizinische Vorrichtung, wobei die erste medizinische Vorrichtung eine Analyt-Messvorrichtung ist; und
b) eine zweite medizinische Vorrichtung, die mit der ersten medizinischen Vorrichtung kommunizieren kann, wobei die zweite medizinische Vorrichtung ein Analyt-Sensor ist,
wobei die erste medizinische Vorrichtung ein Modul zur Kommunikation mit der zweiten medizinischen Vorrichtung umfasst, wobei das Modul zur Kommunikation in der ersten medizinischen Vorrichtung angepasst ist, um durch einen Wert von einer Analyt-Konzentration eines Tieres aktiviert zu werden, wobei die Erzeugung des Wertes der Analyt-Konzentration die Kommunikation zwischen den zwei Vorrichtungen für ein spezifiziertes Zeitlimit aktiviert und Daten von der zweiten medizinischen Vorrichtung zu der ersten medizinischen Vorrichtung übertragen werden können und/oder Befehle von der ersten medizinischen Vorrichtung zu der zweiten medizinischen Vorrichtung gesendet werden können, wobei nach einem Ablauf des Zeitlimits die Kommunikationsverbindung zwischen den zwei Vorrichtungen deaktiviert wird,
wobei zum Aufbau einer weiteren Kommunikation die Kommunikationsverbindung zwischen den zwei Vorrichtungen aktiviert werden muss, indem ein weiterer Wert von einer Analyt-Konzentration des Tieres erzeugt wird, und wobei die erste medizinische Vorrichtung konfiguriert ist, um Befehle an die zweite medizinische Vorrichtung zu senden, um die zweite medizinische Vorrichtung zumindest teilweise zu steuern.

2. System nach Anspruch 1, wobei das Modul zur Kommunikation ein Telemetriesystem zur drahtlosen Kommunikation ist, bevorzugt ein Telemetriesystem zur RF-Kommunikation.

3. System nach Anspruch 1 oder 2, wobei die erste medizinische Vorrichtung ausgewählt ist aus der Gruppe, bestehend aus einer Glukose-Messvorrichtung und einer Streifen-basierten Glukose-Messvorrichtung.

4. System nach Anspruch 1, wobei die Analyt-Konzentration eine Blut-Glukose-Konzentration ist.

5. System nach Anspruch 4, wobei die erste medizinische Vorrichtung ein elektrochemisches Modul zum Messen von Blut-Glukose umfasst.

6. System nach Anspruch 4, wobei die erste medizinische Vorrichtung ein photometrisches Modul zum Messen von Blutkonzentration umfasst.

7. System nach Anspruch 1, wobei die zweite medizinische Vorrichtung ausgewählt ist aus der Gruppe, bestehend aus einem kontinuierlichen Analyt-Sensor und einem kontinuierlichen Glukose-Sensor.

8. System nach Anspruch 1 oder 7, wobei die erste medizinische Vorrichtung und die zweite medizinische Vorrichtung ein Telemetriesystem zur drahtlosen Kommunikation umfassen, bevorzugt ein Telemetriesystem zur RF-Kommunikation.

9. Verfahren zur Kommunikation zwischen einer ersten medizinischen Vorrichtung und einer zweiten medizinischen Vorrichtung, wobei die erste medizinische Vorrichtung eine Analyt-Messvorrichtung ist, wobei die zweite medizinische Vorrichtung ein Analyt-Sensor ist, wobei die erste medizinische Vorrichtung ein Modul zur Kommunikation mit der zweiten medizinischen Vorrichtung umfasst, wobei das Modul zur Kommunikation in der ersten medizinischen Vorrichtung angepasst ist, um durch einen Wert von einer Analyt-Konzentration eines Tieres aktiviert zu werden, wobei die Erzeugung des Wertes von der Analyt-Konzentration des Tieres die Kommunikationsverbindung zwischen den zwei Vorrichtungen für ein spezifiziertes Zeitlimit aktiviert und ermöglicht, dass Daten von der zweiten medizinischen Vorrichtung an die erste medizinische Vorrichtung übertragen werden, und/oder Befehle von der ersten medizinischen Vorrichtung an die zweite medizinische Vorrichtung gesendet werden können, wobei die Kommunikationsverbindung zwischen den zwei Vorrichtungen nach einem Ablauf des Zeitlimits deaktiviert wird, wobei zum Aufbau einer weiteren Kommunikation die Kommunikationsverbindung zwischen den zwei Vorrichtungen aktiviert werden muss, indem ein weiterer Wert von einer Analyt-Konzentration des Tieres erzeugt wird, und wobei die erste medizinische Vorrichtung Befehle an die zweite medizinische Vorrichtung sendet, um die zweite medizinische Vorrichtung zumindest teilweise zu steuern.

10. Verfahren nach Anspruch 9, wobei das Zeitfenster nach einem Wert einer physiologischen Eigenschaft eines Tieres oder nach einem während vorherigen Zeitdauern erlangten Datensatz festgesetzt wird.

11. Verfahren nach den Ansprüchen 9 oder 10, wobei die erste medizinische Vorrichtung ausgewählt ist aus der Gruppe, bestehend aus einer Glukose-Messvorrichtung und einer Streifen-basierten Glukose-Messvorrichtung.

12. Verfahren nach einem der Ansprüche 9 bis 11, wobei die zweite medizinische Vorrichtung ausgewählt ist aus der Gruppe, bestehend aus einem kontinuierlichen Analyt-Sensor und einem kontinuierlichen Glukose-Sensor.

13. Verfahren nach einem der Ansprüche 9 bis 12, wobei die Kommunikation zwischen den medizinischen Vorrichtungen eine drahtlose Kommunikation ist, bevorzugt eine RF-Kommunikation.

## Revendications

1. Système de dispositifs médicaux comprenant :
a) un premier dispositif médical, le premier dispositif médical étant un dispositif de mesure d'analyte et
b) un second dispositif médical capable de communiquer avec ledit premier dispositif médical, dans lequel le second dispositif médical est un capteur d'analyte,
dans lequel ledit premier dispositif médical comprend un module de communication avec le second dispositif médical, dans lequel le module de communication du premier dispositif médical est adapté pour être activé par une valeur d'une concentration d'analyte d'un animal, dans lequel la génération de la valeur de la concentration d'analyte active la communication entre les deux dispositifs pendant une limite de temps spécifiée et des données peuvent être transférées du second dispositif médical au premier dispositif médical et/ou des commandes venant du premier dispositif médical peuvent être envoyées au second dispositif médical, dans lequel, après expiration de la limite de temps, le lien de communication entre les deux dispositifs est désactivé, dans lequel, pour établir une autre communication, le lien de communication entre les deux dispositifs doit être activé pour générer une autre valeur d'une concentration d'analyte de l'animal, et dans lequel le premier dispositif médical est configuré pour envoyer des commandes au second dispositif médical afin de commander au moins en partie le second dispositif médical.

2. Système selon la revendication 1, dans lequel le module de communication est un système de télémesure pour une communication sans fil, de préférence un système de télémesure pour une communication RF.

3. Système selon la revendication 1 ou 2, dans lequel le premier dispositif médical est sélectionné dans le groupe constitué d'un dispositif de mesure du glucose et d'un dispositif de mesure du glucose sur bande.

4. Système selon la revendication 1, dans lequel la concentration d'analyte est une concentration de la glycémie.

5. Système selon la revendication 4, dans lequel le premier dispositif médical comprend un module électrochimique pour mesurer la glycémie.

6. Système selon la revendication 4, dans lequel le premier dispositif médical comprend un module photométrique pour mesurer la glycémie.

7. Système selon la revendication 1, dans lequel le second dispositif médical est sélectionné dans le groupe constitué d'un capteur d'analyte continu et d'un capteur de glucose continu.

8. Système selon la revendication 1 ou 7, dans lequel le premier dispositif médical et le second dispositif médical comprennent un système de télémesure de communication sans fil, de préférence un système de télémesure de communication RF.

9. Procédé de communication entre un premier dispositif médical et un second dispositif médical, le premier dispositif médical étant un dispositif de mesure d'analyte, dans lequel le second dispositif médical est un capteur d'analyte, dans lequel ledit premier dispositif médical comprend un module de communication avec le second dispositif médical, dans lequel le module de communication du premier dispositif médical est adapté pour être activé par une valeur d'une concentration d'analyte d'un animal, dans lequel la génération de la valeur de la concentration d'analyte de l'animal active le lien de communication entre les deux dispositifs pendant une limite de temps spécifiée et permet de transférer des données du second dispositif médical au premier dispositif médical et/ou des commandes venant du premier dispositif médical peuvent être envoyées au second dispositif médical, dans lequel, après expiration de la limite de temps, le lien de communication entre les deux dispositifs est désactivé, dans lequel, pour établir une autre communication, le lien de communication entre les deux dispositifs doit être activé pour générer une autre valeur d'une concentration d'analyte de l'animal, et dans lequel le premier dispositif médical est configuré pour envoyer des commandes au second dispositif médical afin de commander au moins en partie le second dispositif médical.

10. Procédé selon la revendication 9, dans lequel la fenêtre temporelle est établie selon une valeur d'une caractéristique physiologique d'un animal ou selon un ensemble de données obtenues au cours de périodes de temps précédentes.

11. Procédé selon les revendications 9 ou 10, dans lequel le premier dispositif médical est sélectionné dans le groupe constitué d'un dispositif de mesure du glucose et d'un dispositif de mesure du glucose sur bande.

12. Procédé selon l'une quelconque des revendications 9 à 11, dans lequel le second dispositif médical est sélectionné dans le groupe constitué d'un capteur d'analyte continu et d'un capteur de glucose continu.

13. Procédé selon l'une quelconque des revendications 9 à 12, dans lequel la communication entre lesdits dispositifs médicaux est une communication sans fil, de préférence une communication RF.
